(19) Europäisches Patentamt
European Patent Office
Office européen des brevets

(11) Veröffentlichungsnummer : **0 351 644 B1**

(12) # EUROPÄISCHE PATENTSCHRIFT

(45) Veröffentlichungstag der Patentschrift : **10.06.92 Patentblatt 92/24**

(51) Int. Cl.[5] : **C07C 25/13,** C07C 17/33

(21) Anmeldenummer : **89112262.4**

(22) Anmeldetag : **05.07.89**

(54) **Verfahren zur Herstellung von Fluorbenzolen.**

(30) Priorität : **16.07.88 DE 3824141**

(43) Veröffentlichungstag der Anmeldung : **24.01.90 Patentblatt 90/04**

(45) Bekanntmachung des Hinweises auf die Patenterteilung : **10.06.92 Patentblatt 92/24**

(84) Benannte Vertragsstaaten : **BE CH DE FR GB IT LI NL**

(56) Entgegenhaltungen : **DE-A- 2 038 272**

(73) Patentinhaber : **HOECHST AKTIENGESELLSCHAFT**
**Postfach 80 03 20**
**W-6230 Frankfurt am Main 80 (DE)**

(72) Erfinder : **Litterer, Heinz, Dr.**
**Hardtstrasse 77**
**W-6208 Bad Schwalbach (DE)**
Erfinder : **Metz, Hans Joachim, Dr.**
**Wilhelmstrasse 57**
**W-6140 Bensheim (DE)**
Erfinder : **Papenfuhs, Theodor, Dr.**
**Heinrich-Bleicher-strasse 40**
**W-6000 Frankfurt am Main 50 (DE)**
Erfinder : **Sistig, Frank Peter, Dr.**
**Junghainzehecken 43**
**W-6238 Hofheim am Taunus (DE)**
Erfinder : **Leupold, Ernst Ingo, Dr.**
**Am Zäunefeld 15**
**W-6392 Neu-Anspach (DE)**

EP 0 351 644 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von gegebenenfalls substituierten Fluorbenzolen durch katalytische Decarbonylierung der entsprechenden Fluorbenzaldehyde.

Nach dem Stand der Technik hat man Fluoraromaten durch Diazotierung von Anilinderivaten, die am Kern Halogen, insbesondere Fluor, und gegebenenfalls $NH_2$ oder $NO_2$ enthalten, nach der Methode von Balz-Schiemann hergestellt (D.T. Flood, Org. Synth. Coll. II, 295 (1943)). So hat man beispielsweise 1,3-Difluorbenzol durch sukzessive Diazotierung von m-Phenylendiamin in wasserfreiem Fluorwasserstoff mit 31 % Ausbeute erhalten (G. Schiemann et al., Ber. 62, 3039 (1929)). Als Alternativsynthese wird die Diazotierung von m-Fluoranilin in Gegenwart von Ammoniumbifluorid, tertiären Aminen und Dimethylsulfoxyd beschrieben. Die Ausbeuten liegen bei 46 - 73 % (US-PS 4 075 252 und 4 096 196).

Ähnlich niedrige Ausbeuten (27 - 40 %) werden bei der Herstellung von 1,4-Difluorbenzol aus p-Phenylendiamin nach Balz-Schiemann erhalten (G. Schiemann et al., Ber. 62, 3039 (1929)). Auch der Zusatz von tertiären Aminen zur Erzielung günstigerer Zersetzungstemperaturen oder die Verwendung von Aryldiazonium hexafluorophosphat ($ArN_2PF_6$) (K.G. Rutherford et al., J. Org. Chem. 26, 5149 (1961)) führt im allgemeinen zu keinem kostengünstigeren Zugang zu halogensubstituierten Fluorbenzolen.

Es bestand daher die Aufgabe, ein Verfahren zu entwickeln, bei dem neben selektiver Reaktionsführung die üblichen Nachteile wie Umgang mit Fluorwasserstoff oder teuren Hilfschemikalien vermieden werden. Zu diesem Zweck geht man nach der Erfindung einen völlig anderen weg.

Gegenstand der vorliegenden Erfindung ist ein Verfahren, nach dem durch katalytische Decarbonylierung von Fluorbenzaldehyden der Formel II (siehe Patentanspruch 1), worin $R^1$, $R^2$ und $R^3$ unabhängig voneinander H, F, Cl und/oder Alkyl mit 1 bis 3 C-Atomen darstellen, jedoch mindestens einer der Reste Fluor und vorzugsweise mindestens einer der Reste $R^1$ und $R^3$ Wasserstoff ist und $S^1$ und $S^2$ unabhängig voneinander H und/oder Reste, die die Elektronendichte am Benzolkern herabsetzen, vorzugsweise H, sind, vorzugsweise in Gegenwart von Wasserstoff, in der Gasphase bei einer Temperatur von 250 bis 600°C an Zeolithkatalysatoren die entsprechenden Fluorbenzole der Formel I (siehe Patentanspruch 1) in sehr guter Ausbeute erhalten werden.

Die bei dem erfindungsgemäßen Verfahren eingesetzten Zeolithe sind vor allem natürliche oder synthetische kristalline Alumosilikate. Grundbausteine dieser Zeolithe sind $SiO_4$– und $AlO_4$–Tetraeder, die über Sauerstoffatome als Brücken verbunden sind und daher regelmäßige Strukturen mit Hohlräumen und Porenöffnungen haben. Es sind jedoch auch Zeolithe geeignet, bei denen ein Teil des Aluminiums bzw. Siliziums durch andere Gitteratome wie Bor, Eisen, Gallium, Germanium, Titan und/oder Zirkon ersetzt ist. Die negativen Ladungen der $AlO_4$–Tetraeder sind z. T. durch Kationen wie $H^+$, $Na^+$, $K^+$, $Ca^{2+}$, $Mg^{2+}$ oder organische Kationen wie $N^+R_4$ und $P^+R_4$ kompensiert.

für das erfindungsgemäße Verfahren sind auch Katalysatoren geeignet, in denen die durch die Synthese eingebrachten Kationen nach üblichen Methoden gegen andere Kationen ausgetauscht worden sind, so daß Produkte mit sehr unterschiedlichen Eigenschaften vorligen (D.W.Breek : Zeolithe Molecular Sieves, 1974, Kapitel 7, S. 540 - 552 und 569 - 571). Auch die Verwendung von Katalysatoren ist möglich, die durch eine thermische Behandlung von solchen Zeolithtypen, die organische Kationen wie $N^+R_4$ oder $P^+R_4$ enthalten, modifiziert wurden.

Bevorzugt werden jedoch solche Zeolithe verwendet, die weiter durch Ionenaustausch mit ein-, zwei- und-/oder dreiwertigen Kationen, vorteilhaft mit $NH_4^+$, $H^+$, $Be^{2+}$, $Mg^{2+}$, $Ca^{2+}$, $La^{3+}$ oder Kationen der Seltenen Erden oder mit Kombinationen dieser Elemente modifiziert und so in eine katalytisch aktive Form übergeführt sind, wobei solche mit $NH_4^+$ und $H^+$ ganz besonders bevorzugt sind.

Für das erfindungsgemäße Verfahren geeignete Zeolithe - dem Fachmann als Adsorbentien oder als Katalysatoren für Kohlenwasserstoffumwandlungen bekannt - sind z. B. die Zeolithe ZSM-5 (US-PS 3 702 886), ZSM-8 (GB-PS 1 334 243), ZSM-11 (US-PS 3 709 979), ZSM-12 (US-PS 3 832 449), ZSM-20 (US-PS 3 972 983), ZSM-21 (US-PS 4 046 859), ZSM-23 (US-PS 4 076 842) und ZSM-48 (EP-OS 0034918) oder Mordenit. Sämtliche dieser Katalysatoren können als solche eingesetzt werden, werden aber bevorzugt in der H-Form verwendet.

Besonders geeignet für das vorliegende Verfahren sind die Zeolithe vom Pentasiltyp, wobei für den Begriff Pentasile die Definition von Kokotailo und Meier ("Pentasil family of high silicon crystalline materials" in Special Publication No. 33 of the Chemical Society, London 1980) zugrundegelegt ist. Das Si/Al-Verhältnis dieser Zeolithe liegt im allgemeinen zwischen 5 und 4 000, vorzugsweise zwischen 10 und 2 000.

Der Aluminiumgehalt der erfindungsgemäß verwendeten Zeolithe kann zum Zwecke einer weitergehenden Modifizierung durch Behandlung mit Mineralsäuren, organischen Säuren oder chelatisierenden Substanzen innerhalb der genannten Grenzen vermindert werden.

Auch Zeolithe mit Faujasitstruktur sowie solche auf der Basis von Aluminiumphosphat bzw. Aluminium-Silizium-Phosphat sind nach dem erfindungsgemäßen Verfahren einsetzbar.

Die Zeolithe werden für die erfindungsgemäße Verwendung vorteilhaft mit Hilfe von Bindern in eine geeignete Anwendungsform, z. B. in Strangform gebracht. Als Binder sind vor allem Oxyde, Hydroxyde oder Hydroxychloride des Aluminiums und die Oxyde des Siliziums, Titans und Zirkons sowie Tonmaterialien geeignet.

Die bevorzugt verwendeten Zeolith-Katalysatoren sind in üblicher Art durch Calcinieren aktiviert, was eine weitere Art der Modifikation darstellt. Gelegentlich ist es vorteilhaft, Ionenaustausch und Calcinierung mehrmals zu wiederholen. Die Calcinierung wird vorzugsweise bei 350 - 700°C durchgeführt. Um die Stabilisierung zu verbessern, ist es manchmal vorteilhaft, die Calcinierung in Gegenwart von Wasserdampf, Ammoniak oder deren Mischungen bei Temperaturen von mindestens 600°C durchzuführen und dabei die Temperatur evtl. bis auf 900°C zu erhöhen.

Die Decarbonylierung wird in der Gasphase durchegeführt, wobei die Reaktionstemperaturen vorzugsweise 300 - 500°C betragen. Vorzugsweise arbeitet man bei Atmosphärendruck bzw. bei dem Staudruck, der sich durch die Förderung der gasförmigen Ausgangsstoffe durch den z. B. fest angeordneten Katalysator ergibt. Die Drücke betragen z. B. von Atmosphärendruck bis 20 bar, vorzugsweise bis 5 bar.

Vorteilhaft werden zusätzlich zu den zu decarbonylierenden Aldehyden Wasserstoff und gegebenenfalls dampfförmige inerte Lösemittel wie aliphatische Kohlenwasserstoffe wie Pentan-, Hexan- oder Heptanfraktionen oder Gase wie Stickstoff oder Kohlendioxyd über den Katalysator geleitet.

Die Belastung (Gewichtseinheit des Ausgangsmaterials je Stunde pro Gewichtseinheit des Katalysators - Dimension $h^{-1}$ - = WHSV = Weight-Hourly-Space-Velocity) liegt vorzugsweise zwischen 0,1 und 10 $h^{-1}$.

Wie sich aus den nachfolgenden Beispielen ergibt, ist nach einiger Zeit eine Regenerierung des Katalysators erforderlich. Diese kann durch kontrolliertes Abbrennen mit sauerstoffhaltigem Gas erfolgen.

Beispiele

1) Ein gemäß US-PS 3 702 886 mit 0,2 Gew.-% Aluminium synthetisierter Zeolith ZSM-5 (Pentasilstruktur) wurde nach einer 14-stündigen thermischen Vorbehandlung im Verhältnis 2 : 1 mit Aluminiumoxyd vermischt, mit 10 Gew.-% einer 2 gew.-%igen Salpetersäure angeteigt und zu 1,4 mm starken Extrudaten verarbeitet. Die erhaltenen Katalysatorstränge wurden anschließend eine Stunde bei 120°C getrocknet und danach 2 Stunden bei 600°C geglüht. Dann wurden die Stränge mehrmals mit einer 10 %igen Ammonsulfatlösung in Kontakt gebracht und so die durch die Synthese vorhandenen Natriumionen gegen $NH_4^+$ erschöpfend ausgetauscht. Durch eine nochmalige thermische Behandlung dieser Stränge bei 600°C erhielt man die Wasserstofform des Zeolithen ZSM-5.

Ein Festbettreaktor von 20 mm Innendurchmesser und 600 mm Länge wurde mit 100 ml des Katalysators beladen und bei Atmosphärendruck und 400°C mit 60 ml 2,4-Difluorbenzaldehyd beschickt. Zur Erniedrigung des Einlaßpartialdrucks wurden zusätzlich 80 l Wasserstoff pro Stunde zudosiert. Die Ergebnisse des Versuchs sind in Tabelle 1 zusammengefaßt. Die Isolierung des bei 83°C siedenden 1,3-Difluorbenzols erfolgte durch einfache Destillation.

Tabelle 1: Herstellung von 1,3-Difluorbenzol durch Decarbonylierung von 2,4-Difluorbenzaldehyd

| Temperatur (°C) | Zusammensetzung des Reaktoraustrags (Gew.-%) 1,3-Difluorbenzol | 2,4-Difluorbenzaldehyd | nach Stunden (h) |
|---|---|---|---|
| 400 | 99,0 | 1,0 | 1 |
| 400 | 99,0 | 1,0 | 3 |
| 400 | 98,0 | 2,0 | 7 |
| 400 | 94,0 | 6,0 | 12 |
| 400 | 81,0 | 19,0 | 18 |

2) Beispiel 1 wurde mit einem H-ZSM-11-Katalysator (Pentasilstruktur) wiederholt. Die Ergebnisse unterscheiden sich nicht von denen mit dem H-ZSM-5-Katalysator.

3) Setzte man an Stelle des in Beispiel 1 verwendeten 2,4-Difluorbenzaldehydes 2-Chlor-6-fluorbenzaldehyd ein und arbeitete im übrigen wie im Beispiel 1, so wurden über einen mehrstündigen Versuchszyklus folgende Werte erhalten:

Tabelle 2: Herstellung von 3-Chlor-fluorbenzol

| Temperatur (°C) (°C) | Gehalt des Reaktoraustrags (Gew.-%) 3-Chlorfluorbenzol | 2-Chlor-6-fluorbenzaldehyd | nach Stunden (h) |
|---|---|---|---|
| 420 | 33,2 | 64,9 | 1 |
| 420 | 30,2 | 69,1 | 3 |
| 420 | 24,6 | 74,9 | 8 |
| 420 | 22,9 | 76,7 | 12 |
| 420 | 21,7 | 77,6 | 20 |

4) Umsetzung von 2,4-Difluorbenzaldehyd an einem H-ZSM-12-Katalysator

Wandelt man die Beispiele 1 bis 3 so ab, daß man an Stelle eines Zeolithen der Serie ZSM-5 oder ZSM-11 die H-Form des Zeolithen ZSM-12 einsetzte und den Festbettreaktor bei Atmosphärendruck mit einer Belastung von 0,6 $h^{-1}$ bei 370°C unter Zusatz von 60 Liter Wasserstoff pro Stunde betrieb, so wurden an diesem Katalysator 73 % des eingesetzten 2,4-Difluorbenzaldehyds umgesetzt. Die Selektivität zu 1,3-Difluorbenzol betrug 98 %.

5) Es wurde ein im Beispiel 3 der EP-OS 0034918 beschriebener Zeolith ZSM-48 synthetisiert und nach bekannten Methoden in die H-Form übergeführt.

In die in Beispiel 1 beschriebene Versuchsapparatur wurden 50 ml H-ZSM-48-Katalysator eingefüllt. Nach Zudosierung von 60 l Wasserstoff pro Stunde bei Atmosphärendruck wurde die Reaktionstemperatur von 450°C im Verlauf von 30 Minuten eingestellt und danach mit einer Belastung von 1,2 $h^{-1}$ 4-Chlor-2-fluorbenzaldehyd zudosiert.

Aus den Analysendaten der in den ersten 6 Betriebsstunden gesammelten Austräge errechnete sich ein Umsatz von 87 %. Die Selektivität zu 3-Chlor-fluorbenzol lag bei 90 %.

**Patentansprüche**

1. Verfahren zur Herstellung von Fluorbenzolen der Formel (I)

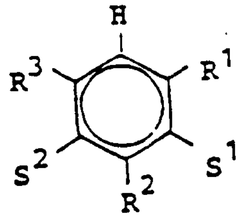

(I),

worin $R^1$, $R^2$ und $R^3$ unabhängig voneinander H, F, Cl und/oder Alkyl mit 1 bis 3 C-Atomen darstellen, jedoch mindestens einer dieser Reste Fluor und vorzugsweise mindestens einer der Reste $R^1$ und $R^3$ Wasserstoff ist

und $S^1$ und $S^2$ unabhängig voneinander H und/oder Reste, die die Elektronendichte am Benzolkern herabsetzen, vorzugsweise H, sind, dadurch gekennzeichnet, daß man Verbindungen der Formel (II)

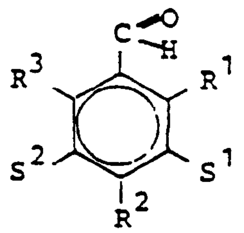

(II),

worin $R^1$, $R^2$, $R^3$, $S^1$ und $S^2$ die vorgenannte Bedeutung haben, vorzugsweise in Gegenwart von Wasserstoff und/oder Stoffen, die gegenüber den Reaktionsteilnehmern unter den angewandten Reaktionsbedingungen inert sind, in der Gasphase bei einer Temperatur von 250 bis 600°C an Zeolithkatalysatoren decarbonyliert.

2. Verfahren nach Anspruch 1, dadurch gekennzeichnet, daß als Zeolith ein Aluminosilikat verwendet wird, das unmodifiziert ist oder bei dem ein Teil des Aluminiums bzw. Siliziums durch Bor, Eisen, Gallium, Germanium, Titan und/oder Zirkon ersetzt ist.

3. Verfahren nach Anspruch 2, dadurch gekennzeichnet, daß das Si/Al-Verhältnis in den Zeolithen zwischen 5 und 4 000, vorzugsweise zwischen 10 und 2 000 liegt.

4. Verfahren nach einem oder mehreren der Ansprüche 1 bis 3, dadurch gekennzeichnet, daß man Zeolithe mit Pentasil- oder Faujasitstruktur oder solche auf der Basis von Aluminiumphosphat oder Aliminium-Silizium-Phosphat verwendet.

5. Verfahren nach einem oder mehreren der Ansprüche 1 bis 4, dadurch gekennzeichnet, daß man zeolith ZSM-5, ZSM-8, ZSM-11, ZSM-12, ZSM-20, ZSM-21, ZSM-23, ZSM-48 in der gewöhnlichen oder H-Form oder Mordenit verwendet.

6. Verfahren nach einem oder mehreren der Ansprüche 1 bis 5, dadurch gekennzeichnet, daß in die Zeolithe durch Ionenaustausch $Be^{2+}$, $Mg^{2+}$, $Ca^{2+}$, $La^{3+}$ und/oder sonstige Kationen oder Seltenen Erden und insbesondere $H^+$ und $NH_4^+$ oder Kombinationen dieser Kationen eingeführt sind.

7. Verfahren nach einem oder mehreren der Ansprüche 1 bis 6, dadurch gekennzeichnet, daß man bei einer Temperatur von 300 bis 500°C arbeitet.

8. Verfahren nach einem oder mehreren der Ansprüche 1 bis 7, dadurch gekennzeichnet, daß man bei einem Druck von Atmosphärendruck bis 20 bar, vorteilhaft bis 5 bar, vorzugsweise bei Atmosphärendruck bzw. bei dem Staudruck, der sich durch die Förderung der gasförmigen Ausgangsstoffe durch das Katalysatorsystem einstellt, arbeitet.

## Claims

1. A process for the preparation of fluorobenzenes of the formula (I)

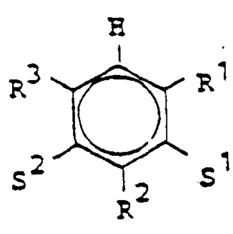

(I),

in which $R^1$, $R^2$ and $R^3$ independently of one another represent H, F, Cl and/or alkyl having 1 to 3 carbon atoms, but at least one of these radicals is fluorine and preferably at least one of the radicals $R^1$ and $R^3$ is hydrogen, and $S^1$ and $S^2$ independently of one another are H and/or radicals which reduce the electron density of the benzene nucleus, preferably H, which comprises decarbonylating a compound of the formula (II)

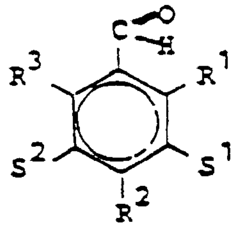

(II),

in which $R^1$, $R^2$, $R^3$, $S^1$ and $S^2$ are as defined above, in the gas phase at a temperature of from 250 to 600°C over zeolite catalysts, preferably in the presence of hydrogen and/or substances which are inert towards the reactants under the reaction conditions applied.

2. The process as claimed in claim 1, wherein the zeolite used is an aluminosilicate which is unmodified or in which a part of the alumino and/or silicon has been replaced by boron, iron, gallium, germanium, titanium and/or zirconium.

3. The process as claimed in claim 2, wherein the Si/Al ratio in the zeolite is between 5 and 4,000, preferably between 10 and 2,000.

4. The process as claimed in one or more of claims 1 to 3, wherein a zeolite having a pentasil structure or faujasite structure or a zeolite based on aluminum phosphate or aluminum-silicon phosphate is used.

5. The process as claimed in one or more of claims 1 to 4, wherein zeolite ZSM-5, ZSM-8, ZSM-11, ZSM-12, ZSM-20, ZSM-21, ZSM-23 or ZSM-48 in the usual form or H-form or mordenite is used.

6. The process as claimed in one or more of claims 1 to 5, wherein $Be^{2+}$, $Mg^{2+}$, $Ca^{2+}$, $La^{3+}$ and/or other cations or rare earths and especially $H^+$ and $NH_4^+$ or combinations of these cations have been introduced into the zeolite by ion exchange.

7. The process as claimed in one or more of claims 1 to 6, which is carried out at a temperature from 300 to 500°C.

8. The process as claimed in one or more of claims 1 to 7, which is carried out at a pressure from atmospheric pressure up to 20 bar, preferably up to 5 bar, preferably at atmospheric pressure or at the back pressure resulting from the passage of the gaseous starting materials through the catalyst system.

**Revendications**

1. Procédé pour préparer des fluorobenzènes répondant à la Formule I :

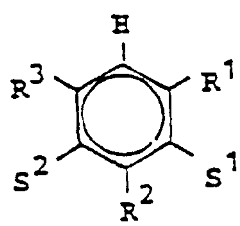

(I)

dans laquelle $R^1$, $R^2$ et $R^3$ représentent chacun, indépendamment l'un de l'autre, H, F, Cl ou un alkyle contenant de 1 à 3 atomes de carbone mais au moins un de ces symboles représente le fluor et, de préférence, au moins un des symboles $R^1$ et $R^3$ représente l'hydrogène, et $S^1$ et $S^2$ représentent chacun, indépendamment l'un de l'autre, H ou un radical abaissant la densité électronique sur le noyau benzénique, de préférence H, procédé caractérisé en ce qu'on décarbonyle des composés répondant à la Formule II :

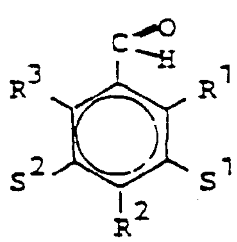

(II)

dans laquelle $R^1$, $R^2$, $R^3$, $S^1$ et $S^2$ ont les significations qui leur ont été données ci-dessus, de préférence en présence d'hydrogène et/ou de corps qui, dans les conditions réactionnelles appliquées, sont inertes à l'égard des composés participant à la réaction, en phase gazeuse, à une température de 250 à 600°C, au contact de zéolites comme catalyseurs.

2. Procédé selon la revendication 1 caractérisé en ce qu'on utilise comme zéolite un aluminosilicate qui n'est pas modifié ou dans lequel une partie de l'aluminium ou du silicium est remplacée par du bore, du fer, du gallium, du germanium, du titane et/ou du zirconium. .

3. Procédé selon la revendication 2 caractérisé en ce que le rapport Si/Al dans les zéolites est compris entre 5 et 4.000, de préférence entre 10 et 2.000.

4. Procédé selon une ou plusieurs des revendications 1 à 3, caractérisé en ce qu'on utilise des zéolites ayant la structure des pentasils ou de la faujasite ou des zéolites à base de phosphate d'aluminium ou de phosphate d'aluminium-silicium.

5. Procédé selon une ou plusieurs des revendications 1 à 4, caractérisé en ce qu'on utilise la zéolite ZSM-5, ZSM-8, ZSM-11, ZSM-12, ZSM-20, ZSM-21, ZSM-23 ou ZSM-48, sous la forme habituelle ou sous la forme H, ou la mordénite.

6. Procédé selon une ou plusieurs des revendications 1 à 5, caractérisé en ce qu'on a introduit dans les zéolites, par échange d'ions, des cations $Be^{2+}$, $Mg^{2+}$, $Ca^{2+}$ ou $La^{3+}$ et/ou d'autres cations ou des terres rares, et plus particulièrement $H^+$ et $NH_4^+$, ou des associations de ces cations.

7. Procédé selon une ou plusieurs des revendications 1 à 6, caractérisé en ce qu'on opère à une température de 300 à 500°C.

8. Procédé selon une ou plusieurs des revendications 1 à 7, caractérisé en ce qu'on opère sous une pression pouvant aller de la pression atmosphérique jusqu'à 20 bar, avantageusement jusqu'à 5 bar, de préférence sous la pression atmosphérique ou sous la pression dynamique qui s'établit par le transport des corps de départ gazeux à travers le système catalytique.